(19) Europäisches Patentamt
European Patent Office
Office européen des brevets

(11) Numéro de publication: **0 007 880 B1**

(12) # FASCICULE DE BREVET EUROPEEN

(45) Date de publication du fascicule du brevet: **20.07.83**

(21) Numéro de dépôt: **79400544.7**

(22) Date de dépôt: **01.08.79**

(51) Int. Cl.³: **C 07 C 147/06**, C 07 C 147/08, C 07 C 147/10, C 07 C 147/12, A 61 K 31/10

(54) **Nouveaux médicaments contenant, à titre de substance active, des composés de type benzènesulfone, nouveaux composés de ce type et procédé pour leur préparation.**

(30) Priorité: **02.08.78 FR 7822842**

(43) Date de publication de la demande: **06.02.80 Bulletin 80/3**

(45) Mention de la délivrance du brevet: **20.07.83 Bulletin 83/29**

(84) Etats contractants désignés: **BE CH DE GB IT NL**

(73) Titulaire: **CHOAY S.A., 48, Avenue Théophile-Gautier, F-75782 Paris Cédex 16 (FR)**

(72) Inventeur: **Loiseau, Philippe, 4, Square d'Angiviller, F-78120 Rambouillet (FR)**
Inventeur: **Fournier, Jean-Paul, 10, rue Fontenay, F-78000 Versailles (FR)**

(74) Mandataire: **Gutmann, Ernest et al, Cabinet Plasseraud 84, rue d'Amsterdam, F-75009 Paris (FR)**

(56) Documents cités:
DE-C- 949 054
FR-A-1 297 775
FR-A-1 442 292
FR-A-2 116 579
FR-A-2 141 749
FR-A-2 240 201
FR-M- 399
GB-A- 544 849
US-A-3 124 447
US-A-3 186 904
US-A-3 699 171

CHEMICAL ABSTRACTS, vol. 42 no. 20, 20 octobre 1948 ref 7882f Columbus, Ohio, USA E. HOGGARTH et al.: «Chemotherapy of tuberculosis. I. Sulfones»
CHEMICAL ABSTRACTS, vol. 72, 1970, page 271, ref 120734a Columbus Ohio, USA M. CINQUINI et al.: «Participation of the sulfinyl oxygen as a neighboring group and solvent effects in the solvolysis of chloroalkyl sulfoxides»
JOURNAL OF ORGANOMETTALIC CHEMISTRY, Vol. 112 (1976) LAUSANNE (CH) A. BONGINI et al.: «Synthesis and reactivity of the 1,1-dilithio derivatives of alkyl phenyl sulfones and N,N-dimethyl methanesulfonamide» pages 1–8
C. R. ACAD. SCI. Serie C, tome 287, 10 juillet 1978 Paris FR R. MOREAU et al.: «Hydroboration de quelques arysulfones beta, gamma-éthyléniques», pages 39–42

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 007 880 B1

ACTORUM AG

# Nouveaux médicaments contenant, à titre de substance active, des composés de type benzènesulfone, nouveaux composés de ce type et procédé pour leur préparation

L'invention est relative à de nouveaux médicaments contenant, à titre de substance active, des composés présentant une structure de base du type benzènesulfone. L'invention est aussi relative aux nouveaux composés servant à l'élaboration de ces nouveaux médicaments. L'invention est enfin relative à un procédé de préparation de ces composés.

Des sulfones du type $ArSO_2R$ dans lesquelles Ar représente un noyau aromatique et R représente un radical alcoyle ont été décrites dans:

les Chemical Abstract, vol. 42, n° 20, (20 octobre 1948), réf. 7882f qui citent par exemple la p-méthoxyphénylisoamylsulfone;

le brevet US n° 3124447 qui concerne des composés dans lesquels R peut représenter un radical aliphatique présentant une chaîne de 1 à 5 atomes de carbone;

le brevet US n° 3186904 qui concerne des composés dans lesquels R peut représenter un radical alkyle;

le brevet FR n° 1442292 qui concerne des composés dans lesquels R peut représenter un groupe alcoyle contenant de préférence jusqu'à 4 atomes de carbone;

le brevet US n° 3699171 qui concerne des composés dans lesquels R représente un radical alkyle ayant de 1 à 20 atomes de carbone;

la demande de brevet FR n° 2240201 qui concerne des composés dans lesquels R peut représenter un alkyle;

les Chemical Abstract, vol. 72, (1970), p. 271, réf. 120734a qui concerne des composés dans lesquels R peut représenter un alcoyle.

Des sulfones du types $ArSO_2R$ dans lesquelles R peut représenter un groupe hydroxyalkyle ayant 2 ou 3 atomes de carbone ont été décrites dans les C.R. Acad. Sci. Paris, t. 287 (10 juillet 1978), série C, p. 39–42.

Par ailleurs, la demande de brevet FR n° 2240201 peut être considérée comme englobant la réaction de la méthylphénylsulfone sur l'acétaldéhyde.

En ce qui concerne les sulfones de type $ArSO_2R$ dans lesquelles R est un radical hydroxyalkyle ayant 4 atomes de carbone ou plus, certaines ont déja été décrites ou suggérées dans les documents antérieurs suivants:

le brevet FR n° 1297775 qui décrit des composés dans lesquels le noyau aromatique est disubstitué par les substituants $NO_2$ et $CH_3$;

la demande de brevet FR n° 2240201 qui peut être considérée comme englobant les réactions d'une des sulfones suivantes: méthylphénylsulfone, éthylphénylsulfone, l'isopropylphénylsulfone, sur l'une des cétones ou l'un des aldéhydes suivants: acétone, méthyléthylcétone, diéthylcétone, acétaldéhyde, aldéhyde propionique et aldéhyde valérique, ainsi que les réactions respectives de la méthylphénylsulfone, l'éthylphénylsulfone, et l'isopropylphénylsulfone sur l'acétophénone, la benzophénone et le benzaldéhyde et qui mentionne également la réaction du cyclohexanone, sur la méthylphénylsulfone, l'éthylphénylsulfone ou l'isopropylphénylsulfone;

le Journal of Organometallic Chemistry, 112 (1976), p. 1–8 qui décrit le 1-benzènesulfonylméthylcyclohexanol ainsi que le (p-chlorophényl)-2-(benzènesulfonyl)éthanol;

les C. R. Acad. Sc. Paris, t. 287 (10 juillet 1978), série C, p. 39–42 qui décrivent le cmposé $ArSO_2R$ dans lesquel Ar représente $p-CH_3-C_6H_5$ et R représente $CH_2-CHOH-C_2H_5$.

Mais l'enseignement de ces documents antérieurs n'indique rien quant à une éventuelle activité pharmacologique des composés qui y sont décrits ou qui y sont suggérés.

Jusqu'á présent, les seules sulfones de type $ArSO_2R$ présentées comme possédant des propriétés pharmacologiques se manifestant par une action sur le système nerveux central, ont un radical R représentant le groupe éthyle.

Il s'agit des parahalogénophényléthylsulfones qui font l'objet du BSM n° 399.

On peut également citer les sulfones du type $ArSO_2-CH_2CH_2OH$ mentionnées dans le brevet DE n° 949054 et pour lesquelles il est indiqué, sans aucune précision, qu'elles peuvent être utilisées comme produits pharmaceutiques.

Enfin, il s'est avéré que des sulfinones ont déjà été testées sur le plan pharmacologique dans le domaine du traitement du dérèglement cardiovasculaire. La formule chimique de ces sulfinones est mentionnée avec celle des sulfones correspondantes dans le brevet FR n° 2116579. Les composés visés dans ce document ont comme formule $ArSO_2R$ dans laquelle Ar est un noyau aromatique et R est soit un radical alkyle ou alcényle, soit un radical hydroxyalkyle, ayant 2 ou 3 atomes de carbone. Par ailleurs, le noyau aromatique de ces composés comporte toujours un, deux ou trois substituants, ces substituants étant dans le cas d'une monosubstitution un radical alcoxy ou alcoyle de 1 à 3 atomes de carbone.

L'enseignement de ce brevet n'apporte donc rien au niveau pharmacologique et thérapeutique sur les structures des sulfones actives.

Les médicaments selon l'invention sont caractérisés en ce qu'ils contiennent, à titre de substance active, au moins un composé de type benzènesulfone répondant à la formule générale

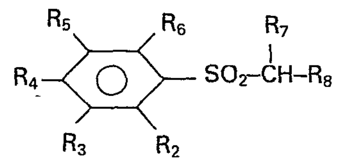

(I)

Dans cette formule, les substituants

$R_2$ à $R_6$ sont indépendamment les uns des autres: un hydrogène, un halogène, un radical $-CH_3$ ou $-OCH_3$, $-NO_2$, $-NH_2$, ou le radical dérivé en substituant un ou deux hydrogènes du groupe

amino par des radicaux alkyle identiques ou différents pouvant comporter de 1 à 3 carbones,

$R_7$ est soit un hydrogène, soit un radical alkyle ou alcényle pouvant avoir jusqu'à 3 carbones,

$R_8$ est un radical hydroxy-alkyle, hydroxy-cycloalkyle, hydroxy-alkyle-aryle, éventuellement substitué sur le noyau aromatique, notamment par un halogène, l'hydroxyle de ceux-ci pouvant être estérifié le cas échéant, étant entendu que:

lorsque $R_8$ est un hydroxyalkyle ayant un nombre d'atomes de carbone égal ou inférieur à 2:

a) le noyau aromatique n'est par disubstitué;

b) le noyau aromatique n'est pas trisubstitué;

c) si le noyau aromatique est monosubstitué, le substituant est différent de $-CH_3$ et de $-OCH_3$;

sous réserve que lorsque $R_8$ représente $-CH_2OH$ et $R_7$ est un atome d'hydrogène, le noyau aromatique soit monosubstitué, le substituant étant différent de $-CH_3$, $-OCH_3$ et d'un atome d'halogène.

Dans une forme préférée, les médicaments selon l'invention contiennent, à titre de substance active, au moins un composé de formule (I) dans laquelle

$R_2$ est $-H$ ou $-OCH_3$,

$R_3$ est $-H$ ou $-OCH_3$,

$R_4$ est $-H$, $-NO_2$, $-NH_2$, $-Cl$, $-F$, $-CH_3$, $-OCH_3$,

$R_5$ est $-H$ ou $-Cl$,

$R_6$ est $-H$,

$R_7$ est $-H$, $-CH_3$, $-C_2H_5$, $-nC_3H_7$.

Dans une autre forme préférée, les médicaments selon l'invention contiennent, à titre de substance active, au moins un composé de formule (I) dans laquelle

$R_8$ est l'un des radicaux: $-CH_2OH$, $-CH_2-CH_2OH$, $-CHOH-CH_3$, $-CHOH-C_2H_5$, $-C(CH_3)_2OH$, $-C(CH_3)(C_2H_5)OH$, $-C_6H_{10}OH$, $-CHOH-C_6H_5$, $-CHOH-C_6H_4Cl$, $-CHOH-C_6H_4F$, $-CHOH-C_6H_3(OCH_3)_2$, $-COH(C_6H_5)_2$, et les esters dérivés: $-CH_2-CH_2-O-CO-C_6H_4NO_2$, $-CH_2O-CO-CH_2-O-C_6H_4Cl$, $-CH_2-CH_2-O-CO-CH_2-O-C_6H_4Cl$, $-CH_2-CH_2-O-CO-C(CH_3)_2-O-C_6H_4Cl$.

Pour former les médicaments selon l'invention, des substances actives préférées sont les composés suivants:

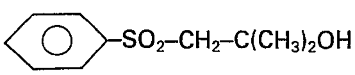

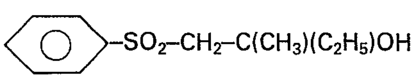

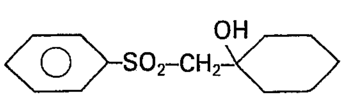

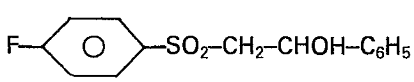

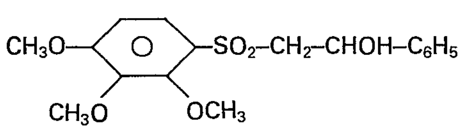

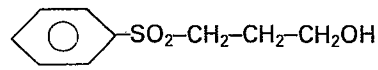

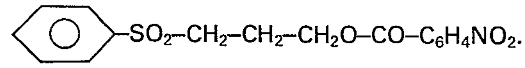

Les médicaments selon l'invention présentent un ensemble de propriétés pharmacologiques et thérapeutiques qui en font des produits de grande valeur.

Les propriétés les plus remarquables se manifestent dans le domaine des troubles de la lipidémie. Ils permettent notamment d'abaisser le taux de lipides circulants chez un sujet présentant une hyperlipidémie. Ces médicaments sont en conséquence particulièrement utiles pour le traitement de troubles de la lipémie tels que l'hypercholestérolémie ou l'hypertriglycéridémie qui précèdent ou accompagnent notamment les maladies athéromateuses.

Les médicaments selon l'invention peuvent aussi agir sur les troubles dans lesquels intervient le système nerveux central. Ils peuvent ainsi manifester des propriétés tranquillisantes, anticonvulsivantes, anti-émétique, anti-ulcérogène, etc.

Aux doses actives, en particulier lorsqu'ils sont utilisés pour leurs propriétés normolipémiantes, les médicaments selon l'invention sont dépourvus de toxicité. Le rapport dose active/dose toxique peut être comparé favorablement à celui de substances connues présentant des propriétés de même nature.

En plus des substances actives constituées par les composés de formule (I), les médicaments selon l'invention peuvent contenir d'autres substances actives compatibles avec les premières.

Dans ces médicaments selon l'invention, les substances actives sont associées, dans la mesure où cela est nécessaire, avec des excipients et adjuvants traditionnels destinés à faciliter et améliorer leur utilisation, leur conservation, etc. En particulier, les substances actives sont associées aux excipients solides ou liquides facilitant leur administration en fonction de la voie d'introduction.

Compte tenu de leurs activités et des traitements pour lesquels ils sont utilisés, les médicaments selon l'invention sont avantageusement administrés par voie parentérale ou par voie orale. Ils peuvent, à cet effet, être présentés sous des formes très variées: comprimés, capsules, gélules, poudres, solutions, suspensions, sirops, suppositoires...

Ils peuvent aussi, en association avec des composés lipidiques, conduire à la formation de présentations pharmaceutiques du type liposome.

Les composés nouveaux selon l'invention, utiles pour la préparation de médicaments tels que décrits précédemment, répondent à la formule générale (I) dans laquelle

$R_7$ est soit un hydrogène, soit un radical alkyle ou alcényle pouvant avoir jusqu'à 3 atomes de carbone,

$R_8$ est un radical hydroxyalkyle dont le nombre d'atomes de carbone est égal ou supérieur à 3, hydroxycycloalkyle, hydroxyalkylaryle éventuellement substitué sur le noyau aromatique, notamment par un halogène, l'hydroxyle de ces radicaux pouvant être estérifié,

$R_2$ à $R_6$ sont, indépendamment les uns des autres, un hydrogène, un halogène, un radical $-CH_3$ ou $-OCH_3$, $-NO_2$ ou $-NH_2$, étant entendu que:

lorsque $R_8$ est un radical hydroxyalkyle et lorsque le noyau est disubstitué, les deux substituants du noyau ne représentent pas simultanément respectivement $-NO_2$ et $-CH_3$, sous réserve des cas particuliers suivants:

a) lorsque $R_8$ représente l'un des radicaux suivants:

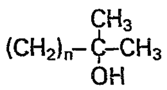
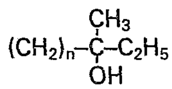
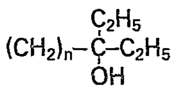
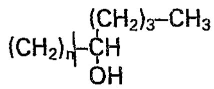

dans lesquels n = 0, 1 ou 2 ou un ester de ceux-ci, l'un au moins des substituants $R_2$ à $R_7$ soit différent de l'hydrogène;

b) lorsque $R_8$ représente le radical:

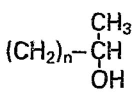 ou 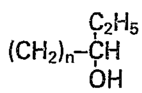

n = 1, 2 ou un ester de celui-ci, l'un au moins des substituants $R_2$ à $R_7$ soit différent de l'hydrogène;

c) lorsque $R_8$ représente le radical:

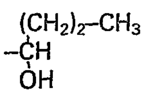

ou un ester de celui-ci, l'un des substituants $R_2$ à $R_7$ soit différent de l'hydrogène;

d) lorsque $R_8$ représente le radical:

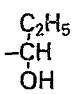

ou un ester de celui-ci, l'un au moins des substituants $R_2$ à $R_7$ soit différent de l'hydrogène et $R_4$ soit différent de $CH_3$;

e) lorsque $R_8$ représente le radical:

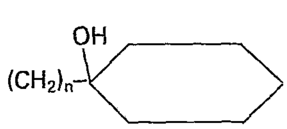

n = 0, 1, 2 ou un ester de celui-ci, l'un au moins des substituants $R_2$ à $R_7$ soit différent de l'hydrogène;

f) lorsque $R_8$ représente l'un des radicaux suivants:

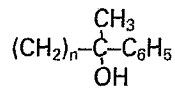 n = 0, 1, 2;

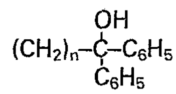 n = 0, 1, 2;

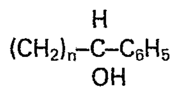 n = 0, 1, 2 et 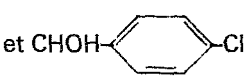

ou un ester de ceux-ci, l'un au moins des substituants $R_2$ à $R_7$ soit différent de l'hydrogène.

Dans un groupe de composés préférés, $R_7$ est soit un hydrogène, soit $-CH_3$, $R_2$, $R_3$, $R_4$, $R_5$, $R_6$ sont indépendamment les uns des autres soit un hydrogène, soit un halogène, soit $-CH_3$, soit $-OCH_3$, $R_8$ est le radical $-CHOH-C_2H_5$, sous réserve que l'un au moins des substituants $R_2$ à $R_7$ soit différent de l'hydrogène et $R_4$ soit différent de $-CH_3$; ou $R_8$ est l'un des radicaux suivants: $-C(CH_3)_2OH$, $-C(CH_3)(C_2H_5)OH$, $-C_6H_{10}OH$, et les esters dérivés de ces composés hydroxylés, sous réserve que l'un au moins des substituants $R_2$ à $R_7$ soit différent de l'hydrogène.

Dans un autre groupe de composés préférés $R_7$ est soit un hydrogène, soit $-CH_3$, $R_2$, $R_3$, $R_4$, $R_5$, $R_6$ sont indépendamment les uns des autres un hydrogène, un halogène, $-CH_3$ ou $-OCH_3$, $R_8$ est l'un des radicaux $-CHOH-C_6H_5$, $-CHOH-C_6H_4Cl$, $-COH(C_6H_5)_2$ sous réserve que l'un au moins des radicaux $R_2$ à $R_7$ soit différent de l'hydrogène ou l'un des radicaux suivants: $-CHOH-C_6H_4F$, $-CHOH-C_6H_3(OCH_3)_2$.

Des composés particulièrement préférés selon l'invention sont ceux de formule:

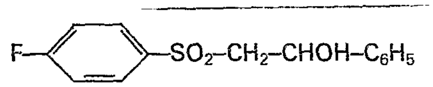

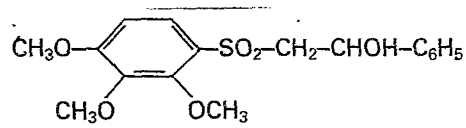

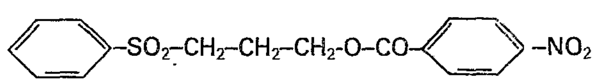

Les composés selon l'invention peuvent être préparés de la façon suivante. A partir de l'acide phényl sulfinique correspondant, on forme en milieu alcalin, par exemple en milieu sodé, le sulfinate. Cette réaction peut être représentée par le schéma

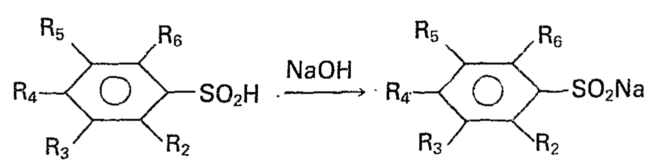


A partir des sulfinates alcalins obtenus, on prépare une sulfone en faisant réagir un agent d'alkylation traditionnel. On peut en particulier, pour introduire un radical alkyle dont le nombre de

carbone ne dépasse pas 3, utiliser un halogénure d'alkyle (RX). Le schéma de cette réaction est:

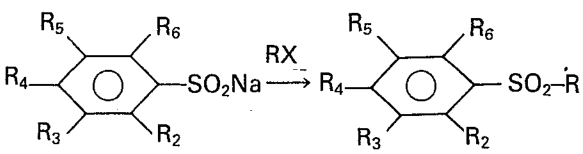


R représente, dans cette formule, l'ensemble $-\overset{R_7}{CH}-R_8$ de la formule (I).

Pour ce type d'alkylation, des halogénures d'alkyle préférés sont: $ICH_3$, $BrCH_2-CH=CH_2$, $IC_2H_5$, $InC_3H7$.

Un mode d'obtention des composés selon l'invention, dans lesquels $R_8$ comprend un hydroxyle, consiste, à partir d'une sulfone correspondante de formule

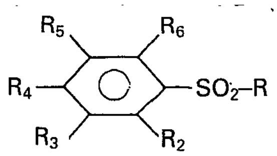

ou encore, pour reprendre la formule générale.

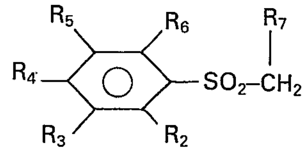

dans laquelle, de préférence, R est $-CH_3$ ou $-C_2H_5$,

soit à former de façon traditionnelle un composé organolithien que l'on fait réagir ensuite avec un composé carbonylé approprié, et à hydrolyser le mélange réactionnel,

soit à former de façon traditionnelle un composé organomagnésien que l'on fait réagir de la même façon avec un composé carbonylé approprié, et à hydrolyser le mélange réactionnel.

Pour former les complexes lithiens ou magnésiens, on utilise de préférence respectivement le butyl-lithium et $C_3H_7MgBr$.

Le schéma réactionnel, si l'on représente le composé carbonylé par O=C–A, est:

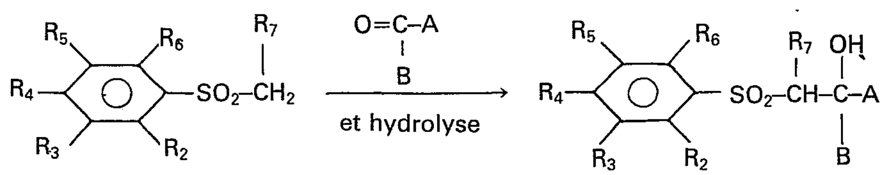


Ce composé correspond à la formule générale (I), $R_8$ étant le radical hydroxylé $-\underset{B}{\overset{OH}{C}}-A$.

Dans cette représentation, A peut être un hydrogène, un radical alkyle, aryle ou alkyle-aryle, B peut être un radical alkyle ou aryle; en outre, A et B peuvent être réunis de façon à former un composé cyclique, notamment dans le cas de la cyclohexanone.

Les exemples suivants illustrent de façon détaillée des modes de préparation des composés de formule générale (I).

Exemple 1

Préparation de l'acide chloro-5 méthoxy-2 nitro-4 benzène-sulfinique de formule

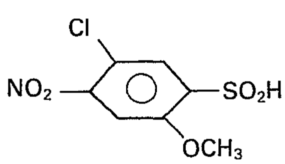

0,2 mole de chlorure de chloro-5 méthoxy-2 nitro-4 benzène-sulfonyle sont agités pendant 2 heures dans une solution de sulfite de sodium, préparée à partir de 0,45 mole de $Na_2SO_3$, $7H_2O$ et de 300 ml d'eau. On maintient le pH alcalin en ajoutant de la soude 5N. La solution de sulfinate est filtrée, acidifiée par l'acide sulfurique dilué. Le précipité d'acide sulfinique est essoré, lavé avec de l'eau glacée et séché. Le rendement est de 72%, PM=251, 63, P.F.=120°C.

Exemple 2

Préparation de la (chloro-5 méthoxy-2 nitro-4 phényl)-méthyl-sulfone et de la (chloro-5 méthoxy-2 amino-4 phényl)méthyl-sulfone de formules respectives

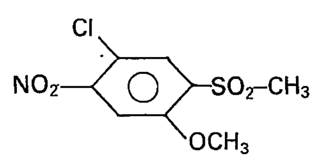
et

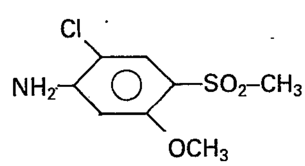

A 0,1 mole (25,2 g) d'une solution dans l'éthanol d'acide chloro-5 méthoxy-2 nitro-4 phényl-sulfinique préparé comme il vient d'être dit, on ajoute 0,1 mole d'éthylate de sodium, puis un excès d'iodure de méthyle. On porte à reflux pendant 6 heures. Après refroidissement, on filtre, on concentre et on cristallise le résidu. Le rendement en sulfone est de 70%, P.F.=148°C.

En variante, la réduction du groupe nitro, par hydrogénation catalytique, conduit au dérivé aminé correspondant.

Le rendement est de 100%; P.F.=150°C.

Exemple 3

Préparation de la (fluoro-4 phényl) [(chloro-4 phényl)-1 hydroxy-1 éthyl]-sulfone de formule

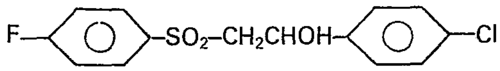

A 0,1 mole (14,7g) de bromure de propyl-magnésium, en solution dans du tétrahydrofurane (T.H.F.) anhydre, on ajoute 0,1 mole (17,4g) de fluoro-4 phényl-méthyl-sulfone, en 15 minutes. On porte à l'ébullition pendant 10 minutes. Après refroidissement, on ajoute 0,10 mole de chloro-4 benzaldéhyde (14,0g) en solution dans du T.H.F. anhydre. Après un contact d'une heure, on porte à reflux. Après refroidissement, on ajoute de l'acide chlorhydrique dilué. On sépare la phase organique et on réextrait plusieurs fois la phase aqueuse. Les extraits oganiques sont réunis, séchés, évaporés. Le résidu est purifié sur colonne d'alumine et cristallisé dans un mélange éther éthylique/cyclohexane. Le rendement est de 92%; P.F.=93°C.

Exemple 4

Préparation du [(chloro-4 phényl) sulfonyl]-2 diphényl-1,1 propanol-1 de formule

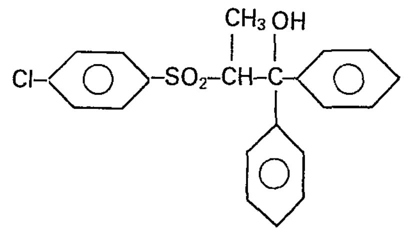

A 0,1 mole de chloro-4 phényl éthylsulfone (20,5g) en solution dans du T.H.F. anhydre, refroidi à −30°C, on ajoute en 30 minutes 64ml de solution 1,6 M de butyl-lithium dans l'hexane. On réchauffe le mélange réactionnel à −5/0°C. Puis on refroidit à −30°C et on ajoute 0,1 mole de benzophénone (18,2g) en solution dans du T.H.F. On laisse revenir à +20°C. On ajoute de l'acide chlorhydrique dilué et on isole le produit comme dans l'exemple 3. Le rendement est de 96%; P.E.$_{15}$=180°C.

Exemples 18 à 41, 44, 46, 47

En suivant les modes de préparation décrits précédemment aux exemples 2, 3, 4, on a préparé une série de composés. Le tableau suivant indique, dans chaque cas, la nature des substituants correspondant à la formule (I), le poids moléculaire du produit, son point de fusion P.F. ou d'ébullition P.E. en degrés C, le rendement et le mode de préparation suivi par référence à l'exemple précédent correspondant.

Hydroxyalkyl-Benzènsulfones

| Ex.n° | $R_2$ | $R_3$ | $R_4$ | $R_5$ | $R_7$ | $R_8$ | P.M. | P.F.°C ou P.E.°C | Rendement% | Méthode de l'ex. |
|---|---|---|---|---|---|---|---|---|---|---|
| 18 | H | H | H | H | H | $-CHOH-CH_3$ | 200,3 | 47 | 95 | 3 |
| 19 | H | H | H | H | H | OH<br>$-C-CH_3$<br>$CH_3$ | 214,2 | $E_{15}$=186 | 60 | 4 |
| 20 | H | H | H | H | H | OH<br>$-C-C_2H_5$<br>$CH_3$ | 228,1 | $E_{16}$=208 | 80 | 4 |
| 21 | H | H | H | H | H | 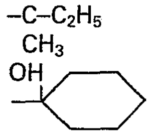 | 254,4 | F=60 | 85 | 4 |
| 22 | H | H | H | H | H | $-CHOH-C_6H_5$ | 262,3 | F=84 | 70 | 4 |
| 23 | H | H | H | H | H | -CHOH- 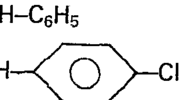 | 296,7 | F=61 | 94 | 3 |
| 24 | H | H | H | H | H | -CHOH- 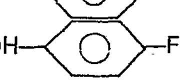 | 280,3 | $E_8$=142 | 86 | 3 |
| 25 | H | H | H | H | $CH_3$ | $-CHOH-C_6H_5$ | 276,3 | $E_1$=143 | 100 | 4 |
| 26 | H | H | H | H | $CH_3$ | -OH<br>$-C-CH_3$<br>$CH_3$ | 228,2 | F=60 | 97 | 4 |
| 27 | H | H | H | H | $CH_3$ | OH<br>$-C-C_6H_5$<br>$C_6H_5$ | 352,4 | F=134 | 95 | 4 |
| 28 | H | H | F | H | H | $-CHOH-C_6H_5$ | 280,3 | F=112 | 73 | 4 |
| 29 | H | H | F | H | H | -CHOH- 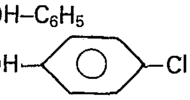 | 314,8 | F=93 | 92 | 3 |

| Ex.n° | $R_2$ | $R_3$ | $R_4$ | $R_5$ | $R_7$ | $R_8$ | P.M. | P.F.°C ou P.E.°C | Rende-ment% | Méthode de l'ex. |
|---|---|---|---|---|---|---|---|---|---|---|
| 30 | H | H | F | H | H | OH<br>$-C-C_2H_5$<br>$CH_3$ | 246,2 | $E_9=164$ | 80 | 3 |
| 31 | H | H | F | H | H | $-CHOH-CH_3$ | 218,2 | F=80 | 92 | 3 |
| 32 | H | H | F | H | H | 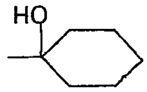 | 272,3 | F=49 | 87 | 3 |
| 33 | $OCH_3$ | $OCH_3$ | $OCH_3$ | H | H | $-CHOH-C_6H_5$ | 252,3 | E=déc. | 75 | 4 |
| 34 | H | H | $CH_3$ | H | H | 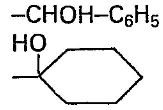 | 268,4 | $E_{10}=180$ | 63 | 3 |
| 35 | H | H | $CH_3$ | H | H | $-CHOH-$ 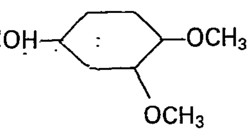 | 336,5 | F=52 | 82 | 3 |
| 36 | H | H | $CH_3$ | H | H | $-CHOH-C_2H_5$ | 228,3 | $E_{0,6}=155$ | 86 | 3 |
| 37 | H | H | Cl | H | $CH_3$ | $-CHOH-C_6H_5$ | 310,8 | $E_8=230$ | 89 | 4 |
| 38 | H | H - | Cl | H | $CH_3$ | OH<br>$-C-C_6H_5$<br>$C_6H_5$ | 386,9 | $E_{15}=180$ | 96 | 4 |
| 39 | H | H | Cl | H | $CH_3$ | 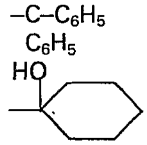 | 302,9 | $E_{11}=195$ | 92 | 4 |
| 40 | H | H | H | H | H | $-CH_2-CH_2OH$ | 200,2 | $E_{17}=240$ | 82 | 4 |
| 41 | H | H | H | H | H | $-CH_2-CH_2O-CO-$ 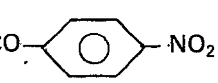 | 249,4 | F=176 | | 4 |
| 44 | $OCH_3$ | H | H | H | H | $-CH_2O-CO-CH_2O-$ 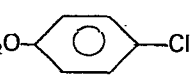 | 384,7 | E=déc. | | 3 |
| 46 | $OCH_3$ | H | H | H | H | $-(CH_2)_2-O-CO-CH_2O-$ 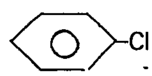 | 398,7 | E=déc. | | 4 |
| 47 | H | H | H | H | H | $-CH_2-CH_2-O-CO-C(CH_2)_2-$ 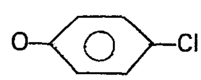 | 396,7 | F=69 | | 4 |

A titre d'exemples, différents essais pour la mise en évidence des propriétés des médicaments selon l'invention sont rapportés ci-après.

a) Toxicité

La détermination est faite sur des souris Swiss de 26 à 30g. Le produit est administré, en solution dans du soluté isotonique, par voie intraveineuse. Le volume administré est de 0,5ml par 20g de poids. Les animaux sont mis en observation pendant 8 jours et la dose létale 50 est calculée suivant la méthode de Behrens et Karber (Arch. Exp. Path. Pharmacol., 1935, 177, 37a).

Les essais effectués ont montré que la dose létale 50 des substances actives était très élevée. Elle s'établit comme suit pour les substances des exemples 23 et 27: 5 animaux sur 5 sont encore en vie après administration par voie intrapéritonéale d'une dose de 1,5g/kg. L'index thérapeutique est donc très favorable.

b) Activité normolipémiante et/ou hypoglycémiante

Les essais sont réalisés sur des groupes de 5 rats Wistar mâles (150 à 175g). Les produits testés sont administrés soit par voie intrapéritonéale, à raison de 100mg/kg dans du soluté isotonique, soit par voie orale, en solution aqueuse à 3% de gomme adragante (1ml/100g de poids corporel) en quantité correspondant à 500mg/kg.

Pour ces essais, on provoque une hyperlipidémie chez les animaux au moyen du produit commercialisé par la Société ROHM et HAAS sous le nom de Triton W.R. Chez le rat à jeun, l'injection intraveineuse de Triton entraine une hyperlipidémie particulièrement nette pour les triglycérides et de façon moindre pour les lipides totaux.

Cette hyperlipidémie est combattue par l'action des substances actives selon l'invention.

Le protocole de l'essai est le suivant. Les rats sont mis à jeûner pendant 18 heures. Le Triton W.R. est administré par voie intraveineuse en solution aqueuse à 10% à raison de 200 mg/kg.

Simultanément, les rats reçoivent les produits à tester. Six heures plus tard, on effectue les dosages de façon traditionnelle sur des échantillons sanguins.

Les résultats de ces essais figurent dans le tableau suivant.

| Produit Exemple n° | Nombre de rats | Glucose g/l | Lipides totaux g/l | Cholestérol Total | Estérifié | Rapport E/T | Triglycérides |
|---|---|---|---|---|---|---|---|
| Témoins | 5 | 0,87 | 6,62 | 0,75 | 0,61 | 79,62 | 1,09 |
| Tém. + Triton | 5 | 0,89 | 13,35 | 1,78 | 1,09 | 65,31 | 3,39 |
| Témoins | 5 | 0,66 | 6,05 | 0,85 | 0,64 | 75,93 | 0,67 |
| Tém. + Triton | 5 | 0,88 | 17,68 | 1,68 | 1,00 | 59,99 | 4,13 |
| 32 | 5 | 0,92 | 10,99 | 1,14 | 0,73 | 65,17 | 4,00 |
| Témoins | 5 | 0,90 | 9,40 | 1,66 | 1,27 | 76,8 | 1,54 |
| Tém. + Triton | 10 | 0,96 | 18,45 | 2,96 | 2,15 | 73,2 | 12,08 |
| 19 | 5 | 0,78 | 7,62 | 2,04 | 1,09 | 55,6 | 2,84 |
| 20 | 5 | 0,84 | 7,36 | 2,20 | 1,64 | 76,8 | 2,07 |
| 21 | 5 | 0,89 | 9,56 | 3,09 | 2,06 | 66,0 | 2,23 |
| 22 | 5 | 1,18 | 8,50 | 2,28 | 1,89 | 82,4 | 3,95 |
| 28 | 5 | 1,22 | 6,0 | 1,24 | 0,66 | 54,4 | 4,31 |
| 33 | 5 | 1,04 | 6,16 | 1,32 | 0,59 | 48,8 | 1,22 |
| 40 | 5 | 0,93 | 3,76 | 1,56 | 0,99 | 61,2 | |
| 41 | 2 | 1,27 | 4,38 | 1,54 | 0,90 | 59,5 | 2,10 |
| Témoins | 5 | 0,74 | 7,90 | 0,85 | 0,62 | 72,96 | 1,32 |
| Tém. + Triton | 5 | 0,90 | 10,20 | 1,55 | 1,01 | 66,13 | 3,47 |
| 24 | 5 | 1,12 | 15,30 | 1,48 | 0,98 | 69,40 | 1,80 |
| Témoins | 5 | 0,79 | 5,89 | 0,50 | 0,39 | 79,67 | 0,74 |
| Tém. + Triton | 5 | 0,85 | 14,26 | 1,58 | 1,03 | 64,48 | 4,63 |
| 47 | 5 | 0,97 | 10,28 | 1,52 | 0,90 | 59,99 | 4,56 |

La comparaison des résultats obtenus avec les témoins ayant reçu le Triton et ceux n'ayant pas réçu de Triton montre l'efficacité des substances actives pour le contrôle de la lipémie.

## Revendications

1. Composé nouveau utile pour la préparation de médicaments, caractérisé en ce qu'il répond à la formule générale suivante:

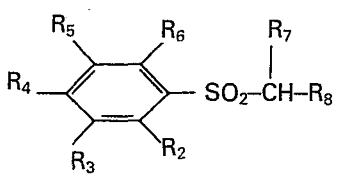

(I)

dans laquelle:

$R_7$ est soit un hydrogène, soit un radical alkyle ou alcényle pouvant avoir jusqu'à 3 atomes de carbone;

$R_8$ est un radical hydroxyalkyle dont le nombre d'atomes de carbone est égal ou supérieur à 3, hydroxycycloalkyle, hydroxyalkylaryle éventuellement substitué sur le noyau aromatique, l'hydroxyle de ces radicaux pouvant être estérifié;

$R_2$ à $R_6$ sont indépendants les uns des autres et représentent un hydrogène, un halogène, un radical $-CH_3$ ou $(OCH_3$, $-NO_2$ ou $-NH_2$, étant entendu que:

lorsque $R_8$ est un radical hydroxyalkyle et lorsque le noyau est disubstitué, les deux substituants du noyau ne représentent pas simultanément respectivement $-NO_2$ et $-CH_3$, sous réserve des cas particuliers suivants:

a) lorsque $R_8$ représente l'un des radicaux suivants:

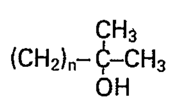

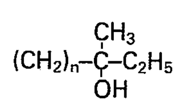

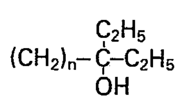

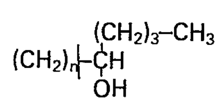

dans lesquels n = 0, 1 ou 2 ou un ester de ceux-ci, l'un au moins des substituants $R_2$ à $R_7$ soit différent de l'hydrogène;

b) lorsque $R_8$ représente le radical:

dans lesquels n = 0, 1 ou 2 ou un ester de ceux-ci, l'un au moins des substituants $R_2$ à $R_7$ soit différent de l'hydrogène;

b) lorsque $R_8$ représente le radical:

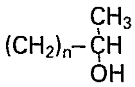

ou

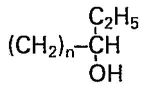

n = 1, 2 ou un ester de celui-ci, l'un au moins des substituants $R_2$ à $R_7$ soit différent de l'hydrogène;

c) lorsque $R_8$ représente le radical:

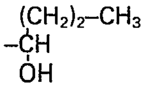

ou un ester de celui-ci, l'un des substituants $R_2$ à $R_7$ soit différent de l'hydrogène;

d) lorsque $R_8$ représente le radical:

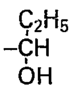

ou un ester de celui-ci, l'un au moins des substituants $R_2$ à $R_7$ soit différent de l'hydrogène et $R_4$ soit différent de $CH_3$;

e) lorsque $R_8$ représente le radical:

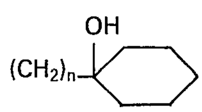

n = 0, 1, 2 ou un ester de celui-ci, l'un au moins des substituants $R_2$ à $R_7$ soit différent de l'hydrogène;

f) lorsque $R_8$ représente l'un des radicaux suivants:

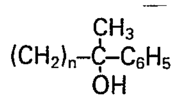 n = 0, 1, 2;

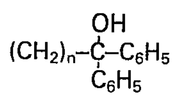 n = 0, 1, 2;

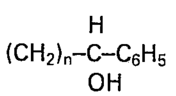 n = 0, 1, 2 et 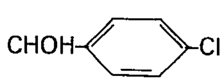

ou un ester de ceux-ci, l'un au moins des substituants $R_2$ à $R_7$ soit différent de l'hydrogène.

2. Composé selon la revendication 1, caractérisé en ce que:

$R_7$ est un hydrogène ou un radical $-CH_3$;

$R_2$, $R_3$, $R_4$, $R_5$, $R_6$ sont indépendamment les uns des autres, soit un hydrogène, soit un halogène, soit $-CH_3$, soit $-CH_3O$;

$R_8$ est le radical $-CHOH-C_2H_5$, sous réserve que l'un au moins des substituants $R_2$ à $R_7$ soit différent de l'hydrogène et $R_4$ soit différent de $CH_3$; ou $R_8$ est l'un des radicaux suivants: $-C(CH_3)_2OH$, $-C(CH_3)(C_2H_5)OH$, $-C_6H_{10}OH$, sous réserve que l'un au moins des substituants $R_2$ à $R_7$ soit différent de l'hydrogène.

3. Composé selon la revendication 1, caractérisé en ce que:

$R_7$ est soit un hydrogène, soit un radical $-CH_3$;

$R_2$, $R_3$, $R_4$, $R_5$, $R_6$ sont, indépendamment les uns des autres, soit un hydrogène, soit un halogène, soit $-CH_3$, soit $-CH_3O$;

$R_8$ est l'un des radicaux suivants: $-CHOH-C_6H_5$, $-CHOH-C_6H_4Cl$, $-COH(C_6H_5)_2$, sous réserve que l'un au moins des substituants $R_2$ à $R_7$ soit différent de l'hydrogène; ou $R_8$ est l'un des radicaux suivants: $-CHOH-C_6H_4F$, $-CHOH-C_6H_3(OCH_3)_2$.

4. Composés selon la revendication 1, caractérisés en ce qu'ils répondent à l'une des formules suivantes:

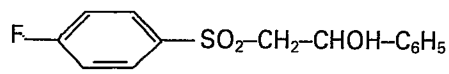

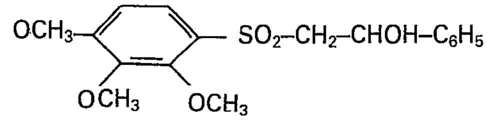

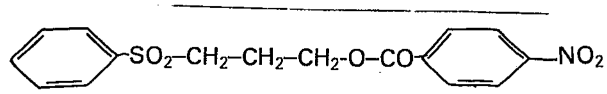

5. Médicament caractérisé en ce qu'il contient à titre de substance active au moins un composé du type benzènesulfone selon l'une quelconque des revendications 1 à 4.

6. Médicament pour le traitement des troubles des lipémies, caractérisé en ce qu'il contient à titre de substance active au moins un composé du type benzènesulfone répondant à la formule générale:

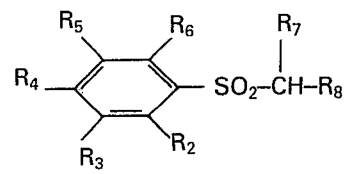 (I)

dans laquelle:

$R_2$ à $R_6$ sont, indépendamment les uns des autres, un hydrogène, un halogène, un radical $-CH_3$ ou $-OCH_3$, $-NO_2$, $-NH_2$, ou le radical dérivé en substituant un ou deux hydrogènes du groupe amino par des radicaux alkyle identiques ou différents pouvant comporter de 1 à 3 atomes de carbone;

$R_7$ est soit un hydrogène, soit un radical alkyle ou alcényle pouvant avoir jusqu'à 3 atomes de carbone;

$R_8$ est un radical hydroxyalkyle, hydroxycycloalkyle, hydroxyalkylaryle éventuellement substitué sur le noyau aromatique, l'hydroxyle de ceux-ci pouvant être estérifié étant entendu que:

lorsque $R_8$ est un hydroxyalkyle ayant un nombre d'atomes de carbone égal ou inférieur à 2:

a) le noyau aromatique n'est pas disubstitué;

b) le noyau aromatique n'est par trisubstitué;

c) si le noyau aromatique est monosubstitué, le

substituant est différent de $-CH_3$ et de $-OCH_3$;

sous réserve que lorsque $R_8$ représente $-CH_2OH$ et $R_7$ est un atome d'hydrogène, le noyau aromatique soit monosubstitué, le substituant étant différent de $-CH_3$, $-OCH_3$ et d'un atome d'halogène.

7. Médicament selon l'une quelconque des revendications 5 ou 6, caractérisé en ce qu'il contient à titre de substance active au moins un des composés de formule (I) dans laquelle:

$R_2$ est $-H$ ou $-OCH_3$,

$R_3$ est $-H$ ou $-OCH_3$,

$R_4$ est $-H$, $-NO_2$, $-NH_2$, $-Cl$, $-F$, $-CH_3$, $-OCH_3$,

$R_5$ est $-H$ ou $-Cl$,

$R_6$ est $-H$,

$R_7$ èst $-H$, $-CH_3$, $-C_2H_5$, $-_nC_3H_7$.

8. Médicament selon l'une quelconque des revendications 5 ou 6, caractérisé en ce qu'il contient à titre de substance active au moins un des composés de formule (I) dans laquelle:

$R_8$ est l'un des radicaux: $-CH_2OH$, $-CH_2-CH_2OH$, $-CHOH-CH_3$, $-CHOH-C_2H_5$, $-C(CH_3)_2OH$, $-C(CH_3)(C_2H_5)OH$, $-C_6H_{10}OH$, $-CHOH-C_6H_5$, $-CHOH-C_6H_4Cl$, $-CHOH-C_6H_4F$, $-CHOH-C_6H_3(OCH_3)_2$, $-COH(C_6H_5)_2$ et les esters dérivés: $-CH_2-CH_2-O-CO-C_6H_4NO_2$, $-CH_2O-CO-CH_2-O-C_6H_4Cl$, $-CH_2-CH_2-O-CO-CH_2-O-C_6H_4Cl$, $-CH_2-CH_2-O-CO-C(CH_3)_2-O-C_6H_4Cl$.

9. Médicaments selon la revendication 6, caractérisés en ce qu'ils répondent à l'une des formules suivantes:

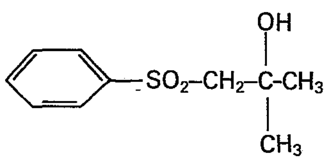

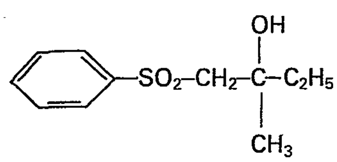

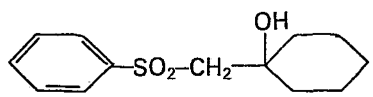

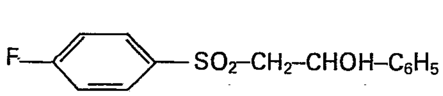

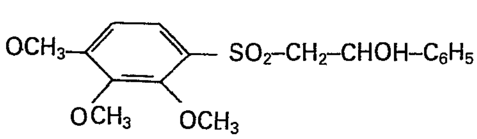

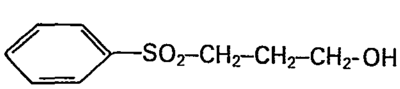

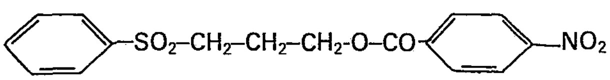

10. Procédé de préparation de composés selon l'une quelconque des revendications 1 à 4, caractérisé en ce qu'à partir de l'acide phénylsulfinique correspondant, on forme en milieu alcalin un sulfinate, puis à partir de celui-ci on prépare, en faisant réagir un agent d'alkylation, une sulfone répondant à la formule suivante:

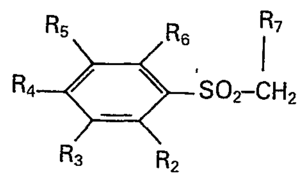

dans laquelle $R_7$ est H ou $CH_3$, à partir de laquelle on forme un dérivé organolithien ou organomagnésien que l'on fait réagir avec un composé carbonylé.

**Patentansprüche**

1. Neue, für die Herstellung von Arzneimitteln nützliche Verbindung, dadurch gekennzeichnet, dass sie der folgenden allgemeinen Formel:

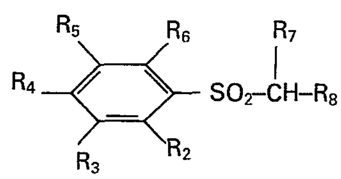

(I)

entspricht, in der:

$R_7$ entweder ein Wasserstoffatom oder eine Alkyl- oder Alkenyl-Gruppe, die bis zu 3 Kohlenstoffatome aufweisen kann, bedeutet;

$R_8$ eine Hydroxyalkylgruppe, deren Anzahl von Kohlenstoffatomen gleich oder grösser als 3 ist, eine Hydroxycycloalkylgruppe oder eine gegebenenfalls am aromatischen Kern substituierte Hydroxyalkylarylgruppe bedeutet, wobei die Hydroxylgruppen dieser Reste verestert sein können;

$R_2$ bis $R_6$ unabhängig voneinander sind und Wasserstoffatome, Halogenatome oder Gruppen der Formeln $-CH_3$, $-OCH_3$, $-NO_2$ oder $-NH_2$ bedeuten, mit der Massgabe, dass

wenn $R_8$ eine Hydroxyalkylgruppe darstellt und der Kern disubstituiert ist, die beiden Substituenten des Kerns nicht gleichzeitig Gruppen der Formeln $-NO_2$ bzw. $-CH_3$ darstellen, und den weiteren Massgaben:

a) wenn R/8 eine Gruppe der folgenden Formeln

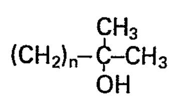

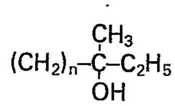

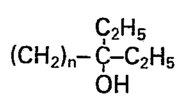

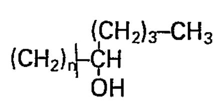

worin n 0, 1 oder 2 darstellt, oder einen Ester dieser Gruppen bedeutet, ist mindestens einer der

Substituenten $R_2$ und $R_7$ von Wasserstoff verschieden;

b) wenn $R_8$ eine Gruppe der Formel

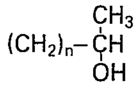 oder 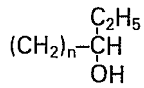

n = 1, 2 oder einen Ester davon darstellt, ist mindestens einer der Substituenten $R_2$ bis $R_7$ von Wasserstoff verschieden;

c) wenn $R_8$ die Gruppe der Formel

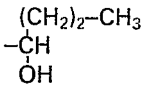

oder einen Ester davon bedeutet, ist einer der Substituenten $R_2$ bis $R_7$ von Wasserstoff verschieden;

d) wenn $R_8$ die Gruppe der Formel

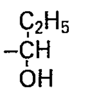

oder einen Ester davon darstellt, ist mindestens einer der Substituenten $R_2$ bis $R_7$ von Wasserstoff und $R_4$ von $-CH_3$ verschieden;

e) wenn $R_8$ eine Gruppe der Formel

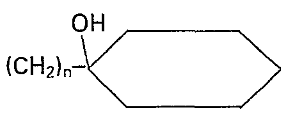

n = 0, 1, 2 oder einen Ester davon bedeutet, ist mindestens einer der Substituenten $R_2$ bis $R_7$ von Wasserstoff verschieden;

f) wenn $R_8$ eine der folgenden Gruppen

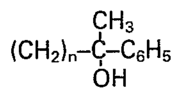 n = 0, 1, 2;

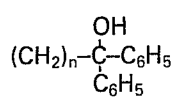 n = 0, 1, 2;

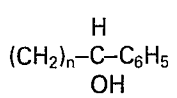 n = 0, 1, 2und 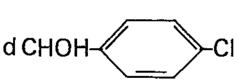

oder einen Ester davon darstellt, ist mindestens einer der Substituenten $R_2$ bis $R_7$ von Wasserstoff verschieden.

2. Verbindung nach Anspruch 1, dadurch gekennzeichnet, dass

$R_7$ ein Wasserstoffatom oder eine Gruppe der Formel $-CH_3$ darstellt;

$R_2$, $R_3$, $R_4$, $R_5$ und $R_6$ unabhängig voneinander sind und entweder Wasserstoffatome oder Halogenatome oder Gruppen der Formeln $-CH_3$ oder $-CH_3O$ bedeuten;

$R_8$ die Gruppe der Formel $-CHOH-C_2H_5$ bedeutet, mit der Massgabe, dass mindestens einer der Substituenten $R_2$ bis $R_7$ von Wasserstoff verschieden ist und $R_4$ von $CH_3$ verschieden ist; oder $R_8$ eine der folgenden Gruppen $-C(CH_3)_2OH$, $-C(CH_3)(C_2H_5)OH$, $-C_6H_{10}OH$ mit der Massgabe bedeutet, dass mindestens einer der Substituenten von $R_2$ bis $R_7$ von Wasserstoff verschieden ist.

3. Verbindung nach Anspruch 1, dadurch gekennzeichnet, dass

$R_7$ entweder ein Wasserstoffatom oder eine Gruppe der Formel $-CH_3$ bedeutet;

$R_2$, $R_3$, $R_4$, $R_5$ und $R_6$ unabhängig voneinander sind und entweder Wasserstoffatome oder Halogenatome oder Gruppen der Formeln $-CH_3$ oder $-CH_3O$ bedeuten;

$R_8$ eine Gruppe der Formeln $-CHOH-C_6H_5$, $-CHOH-C_6H_4Cl$, $-COH(C_6H_5)_2$ mit der Massgabe bedeutet, dass mindestens einer der Substituenten $R_2$ bis $R_7$ von Wasserstoff verschieden ist; oder $R_8$ eine Gruppe der Formeln $-CHOH-C_6H_4F$, $-CHOH-C_6H_3(OCH_3)_2$ darstellt.

4. Verbindungen nach Anspruch 1, dadurch gekennzeichnet, dass sie einer der folgenden Formeln entsprechen:

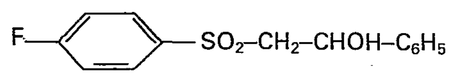

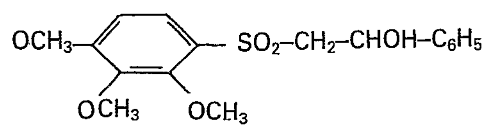

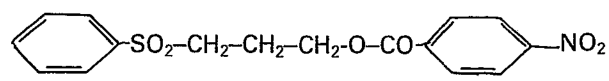

5. Arzneimittel, dadurch gekennzeichnet, dass es als Wirkstoff mindestens eine Verbindung des Benzolsulfontyps gemäss einem der Ansprüche 1 bis 4 enthält.

6. Arzneimittel zur Behandlung von lipämischen Störungen, dadurch gekennzeichnet, dass es als Wirkstoff mindestens eine Verbindung des Benzolsulfontyps der allgemeinen Formel

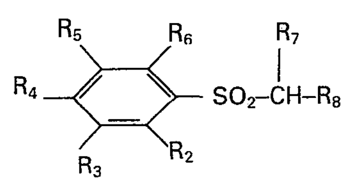 (I)

enthält, in der:

$R_2$ bis $R_6$ unabhängig voneinander Wasserstoffatome, Halogenatome, Gruppen der Formeln $-CH_3$, $-OCH_3$, $-NO_2$ oder $-NH_2$ oder eine Gruppe bedeuten, die durch Substituieren eines oder zweier Wasserstoffatome der Aminogruppe durch gleichartige oder verschiedene Alkylgruppen mit 1 bis 3 Kohlenstoffatomen gebildet wird;

$R_7$ entweder ein Wasserstoffatom oder eine Alkyl- oder Alkenylgruppe mit bis zu 3 Kohlenstoffatomen darstellt;

$R_8$ eine Hydroxyalkylgruppe, eine Hydroxycycloalkylgruppe oder eine gegebenenfalls am aromatischen Kern substituierte Hydroxyalkylarylgruppe bedeutet, wobei die Hydroxylgruppe dieser Reste verestert sein können; mit der Massgabe, dass:

wenn $R_8$ eine Hydroxyalkylgruppe mit 2 oder weniger als 2 Kohlenstoffatomen darstellt:

a) der aromatische Kern nicht disubstituiert ist;

b) der aromatische Kern nicht disubstituiert ist;

c) wenn der aromatische Kern monosubstituiert ist, der Substituent von einer Gruppe der Formeln $-CH_3$ und $-OCH_3$ verschieden ist;

und der weiteren Massgabe, dass wenn $R_8$ eine Gruppe der Formel $-CH_2OH$ und $R_7$ ein Wasserstoffatom darstellen, der aromatische Kern monosubstituiert ist, wobei der Substituent von Gruppen der Formeln $-CH_3$ und $-OCH_3$ und Halogenatomen verschieden ist.

7. Arzneimittel nach einem der Ansprüche 5 oder 6, dadurch gekennzeichnet, dass es als Wirkstoff mindestens eine Verbindung der Formel (I) enthält, in der:

$R_2$ -H oder $-OCH_3$,

$R_3$ -H der $-OCH_3$,

$R_4$ -H, $-NO_2$, $-NH_2$, -Cl, -F, $-CH_3$, $-OCH_3$,

$R_5$ -H oder -Cl,

$R_6$ -H und

$R_7$ -H, $-CH_3$, $-C_2H_5$ oder $-nC_3H_7$ bedeuten.

8. Arzneimittel nach einem der Ansprüche 5 oder 6, dadurch gekennzeichnet, dass es als Wirkstoff mindestens eine Verbindung der Formel (I) enthält, in der

$R_8$ eine der Gruppen der Formeln: $-CH_2OH$, $-CH_2-CH_2OH$, $-CHOH-CH_3$, $-CHOH-C_2H_5$, $-C(CH_3)_2OH$, $-C(CH_3)(C_2H_5)OH$, $-C_6H_{10}OH$, $-CHOH-C_6H_5$, $-CHOH-C_6H_4Cl$, $-CHOH-C_6H_4F$, $-CHOH-C_6H_3(OCH_3)_2$, $-COH(C_6H_5)_2$ oder der davon abgeleiteten Estergruppen: $-CH_2-CH_2-O-CO-C_6H_4NO_2$, $-CH_2O-CO-CH_2-O-C_6H_4Cl$, $-CH_2-CH_2-O-CO-CH_2-O-C_6H_4Cl$, $-CH_2-CH_2-O-CO-C(CH_3)_2-O-C_6H_4Cl$ bedeutet.

9. Arzneimittel nach Anspruch 6, dadurch gekennzeichnet, dass die Wirkstoffe einer der folgenden Formeln entsprechen:

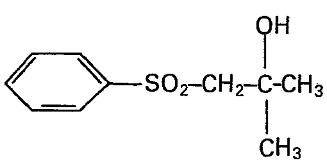

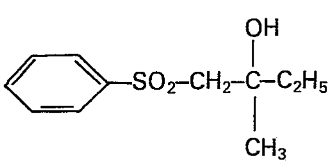

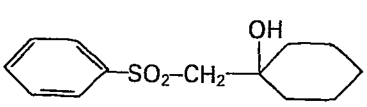

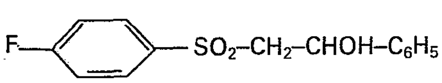

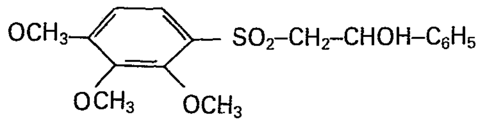

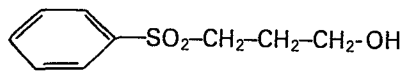

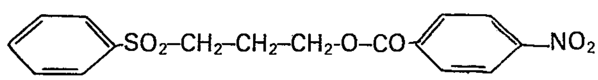

10. Verfahren zur Herstellung der Verbindungen nach einem der Ansprüche 1 bis 4, dadurch gekennzeichnet, dass man ausgehend von der entsprechenden Phenylsulfinsäure in alkalischem Medium ein Sulfinat bildet, dann ausgehend von dieser Verbindung durch Umsetzung mit einem Alkylierungsmittel ein Sulfon der folgenden Formel

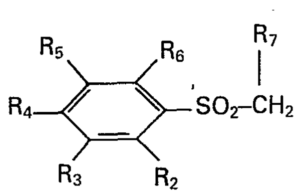

bildet, in der $R_7$ H oder $CH_3$ darstellt, aus der man ein lithiumorganisches oder magnesiumorganisches Derivat bildet, das man mit einer Carbonylverbindung umsetzt.

**Claims**

1. New compound useful for preparing medicaments characterized by the fact that they present the following formula:

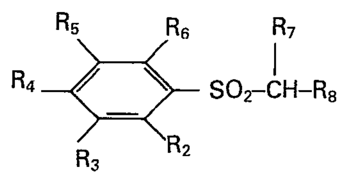

(I)

in which:

$R_7$ is either a hydrogen atom or an alkyl or alcenyl radical which can have up to 3 carbon atoms;

$R_8$ is a hydroxyalkyl radical, the carbon atom number of which is equal or higher than 3, hydroxycycloalkyl, hydroxyalkylaryl radical possibly substituted on the aromatic ring, the hydroxyl group of these radicals being able to be esterified;

$R_2$ to $R_6$ are independent from each others and represent a hydrogen atom, a halogen, a $-CH_3$ or $-OCH_3$, $-NO_2$ or $-NH_2$ radical, with the proviso that:

when $R_8$ is a hydroxyalkyl radical and when the ring is disubstituted, the two substituents of the ring cannot simultaneously represent respectively

$-NO_2$ and $-CH_3$, with the proviso for the following particular cases:

a) when $R_8$ represents one of the following radicals:

$$(CH_2)_n-\overset{CH_3}{\underset{OH}{C}}-CH_3 \qquad (CH_2)_n-\overset{CH_3}{\underset{OH}{C}}-C_2H_5$$

$$(CH_2)_n-\overset{C_2H_5}{\underset{OH}{C}}-C_2H_5 \qquad (CH_2)_n-\overset{(CH_2)_3-CH_3}{\underset{OH}{CH}}$$

in which n = 0, 1 or 2 or an ester thereof, one at least of the substituents $R_2$ to $R_7$ is different from hydrogen;

b) when $R_8$ represents the radical:

$$(CH_2)_n-\overset{CH_3}{\underset{OH}{CH}} \quad \text{ou} \quad (CH_2)_n-\overset{C_2H_5}{\underset{OH}{CH}}$$

n = 1, 2 or an ester thereof, one at least of the substituents $R_2$ to $R_7$ is different from hydrogen;

c) when $R_8$ represents the radical:

$$-\overset{(CH_2)_2-CH_3}{\underset{OH}{CH}}$$

or an ester thereof, one at least of the substituents $R_2$ to $R_7$ is different from hydrogen;

d) when $R_8$ represents the radical:

$$-\overset{C_2H_5}{\underset{OH}{CH}}$$

or an ester thereof, one at least of the substituents $R_2$ to $R_7$ is different from hydrogen and $R_4$ is different from $-CH_3$;

e) when $R_8$ represents the radical:

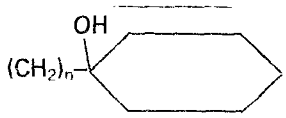

n = 0, 1, 2 or an ester thereof, one at least of the substituents $R_2$ to $R_7$ is different from hydrogen;

f) when $R_8$ represents one of the following radicals:

$$(CH_2)_n-\overset{CH_3}{\underset{OH}{C}}-C_6H_5 \quad n = 0, 1, 2;$$

$$(CH_2)_n-\overset{OH}{\underset{C_6H_5}{C}}-C_6H_5 \quad n = 0, 1, 2;$$

$$(CH_2)_n-\overset{H}{\underset{OH}{C}}-C_6H_5 \quad n = 0, 1, 2 \text{ and CHOH}$$ 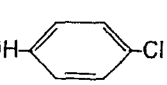

or an ester thereof, one at least of the substituents $R_2$ to $R_7$ is different from hydrogen.

2. Compound according to claim 1, characterized by the fact that:

$R_7$ is a hydrogen or a $-CH_3$ radical;

$R_2$, $R_3$, $R_4$, $R_5$, $R_6$ are inependently from each others, either a hydrogen, or a halogen, or $-CH_3$, or $-CH_3O$;

$R_8$ is the $-CHOH-C_2H_5$ radical, with the proviso that one at least of the substituents $R_2$ to $R_7$ is different from hydrogen and $R_4$ is different from $CH_3$; or $R_8$ is one of the following radicals: $-C(CH_3)_2OH$, $-C(CH_3)(C_2H_5)OH$, $-C_6H_{10}OH$, with the proviso that one at least of the substituents $R_2$ to $R_7$ is different from hydrogen.

3. Compound according to claim 1, characterized by the fact that:

$R_7$ is either a hydrogen, or a $-CH_3$ radical;

$R_2$, $R_3$, $R_4$, $R_5$, $R_6$ are, independently from each others, either a hydrogen, or a halogen, or $-CH_3$ or $-CH_3O$;

$R_8$ is one of the following radicals: $-CHOH-C_6H_5$, $-CHOH-C_6H_4Cl$, $-COH(C_6H_5)_2$, with the proviso that one at least of the substituents $R_2$ to $R_7$ is different from hydrogen; or $R_8$ is one of the following radicals: $-CHOH-C_6H_4F$, $-CHOH-C_6H_3(OCH_3)_2$.

4. Compounds according to claim 1, characterized by the fact that they correspond to one of the following formule:

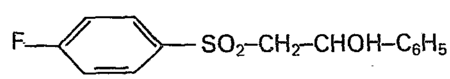

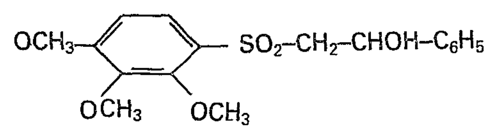

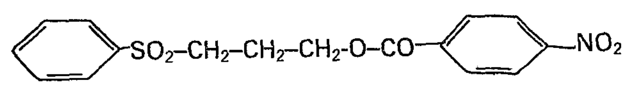

5. Medicament characterized by the fact that it contains as an active substance one at least of the benzenesulfone type compounds according to any of claims 1 to 4.

6. Medicament for treating lipidemia disorders, characterized by the fact that it contains as an active substance one at least of the benzenesulfone type compounds of the following formula:

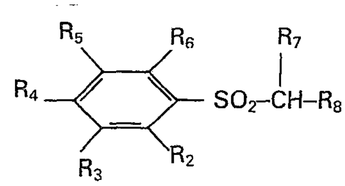 (I)

in which:

$R_2$ to $R_6$ are, independently from each others, a hydrogen, a halogen, a $-CH_3$ or $-OCH_3$ radical, $-NO_2$, $-NH_2$, or the radical derived by substituting one or two hydrogens of the amino group by identical or different alkyl radicals which can comprise from 1 to 3 carbon atoms;

$R_7$ is either a hydrogen, or an alkyl or alcenyl radical which can comprise up to 3 carbon atoms;

$R_8$ is a hydroxyalkyl, hydroxycycloalkyl, hydroxyalkylaryl radical possibly substituted on the aromatic ring, the hydroxyl group of these radicals being able to be esterified with the proviso that:

when $R_8$ is a hydroxyalkyl group with a number of carbon atoms equal or lower than 2:

a) the aromatic ring is not disubstituted;

b) the aromatic ring is not trisubstituted;

c) if the aromatic ring is monosubstituted, the substituent is different from $-CH_3$ and $-OCH_3$; with the proviso that when $R_8$ represents $-CH_2OH$ and $R_7$ is a hydrogen atom, the aromatic ring is monosubstituted, the substituent being different from $-CH_3$, $-OCH_3$ and a halogen atom.

7. Medicament according to claim 5 or 6, characterized by the fact that it contains as an active substance at least one the compounds of formula (I) in which:

$R_2$ is $-H$ or $-OCH_3$,
$R_3$ is $-H$ or $-OCH_3$,
$R_4$ is $-H$, $-NO_2$, $-NH_2$, $-Cl$, $-F$, $-CH_3$, $-OCH_3$,
$R_5$ is $-H$ or $-Cl$,
$R_6$ is $-H$,
$R_7$ is $-H$, $-CH_3$, $-C_2H_5$, $-{}_nC_3H_7$.

8. Medicament according to claim 5 or 6, characterized by the fact that it contains as an active substance at least one of the compounds of formula (I) in which:

$R_8$ is one of the radicals: $-CH_2OH$, $-CH_2-CH_2OH$, $-CHOH-CH_3$, $-CHOH-C_2H_5$, $-C(CH_3)_2OH$, $-C(CH_3)(C_2H_5)OH$, $-C_6H_{10}OH$, $-CHOH-C_6H_5$, $-CHOH-C_6H_4Cl$, $-CHOH-C_6H_4F$, $-CHOH-C_6H_3(OCH_3)_2$, $-COH(C_6H_5)_2$ and the derived esters: $-CH_2-CH_2-O-CO-C_6H_4NO_2$, $-CH_2O-CO-CH_2-O-C_6H_4Cl$, $-CH_2-CH_2-O-CO-CH_2-O-C_6H_4Cl$, $-CH_2-CH_2-O-CO-C(CH_3)_2-O-C_6H_4Cl$.

9. Medicaments according to claim 6, characterized by the fact that they correspond to one of the following formulae:

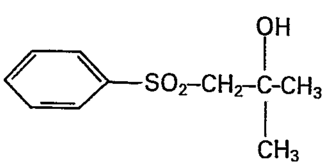

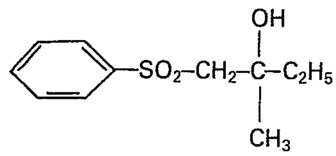

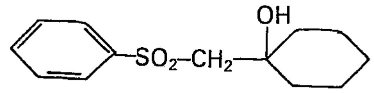

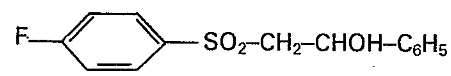

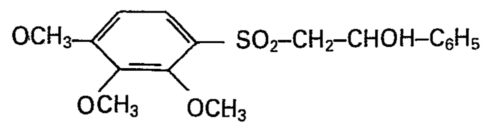

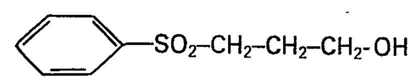

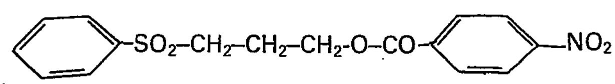

10. Process for preparing compounds according to any of claims 1 to 4, characterized by the fact that from the corresponding phenylsulfinic acid, a sulfinate is prepared in an alkali medium, then from this one, by reacting an alkylating agent, a sulfone which corresponds to the following formula:

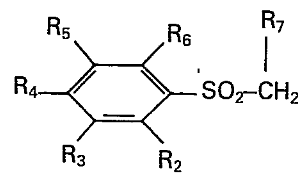

in which $R_7$ is H or $CH_3$, is prepared, from which an organolithien or organomagnesien derivate is prepared which can be reacted with the carbonylated compound.